# EUROPEAN PATENT APPLICATION

(11) **EP 1 872 668 A1**
(43) Date of publication of application: **02.01.2008**
(21) Application number: 05730500.5
(22) Date of filing: 13.04.2005
(51) Int. Cl.: A23L 1/302, A23K 1/16, A23K 1/175, A23L 1/30, A23L 1/304, A23L 1/305, A61K 31/375, A61K 33/26, A61K 38/17, A61P 17/00, A61P 19/00

(54) **NUTRIENT COMPOSITION**

(71) Applicant: SNOW BRAND MILK PRODUCTS, CO., LTD., Sapporo-shi Hokkaido 065-0043 (JP)
(72) Inventor: KATO, Ken, Snow Brand Milk Products Co., Ltd., Kawagoe-shi, Saitama; 3501165 (JP); UEDA, Noriko, Snow Brand Milk Products Co., Ltd., Kawagoe-shi, Saitama 3501165 (JP); TANAKA, Miyako, Snow Brand Milk Products Co., Ltd., Kawagoe-shi, Saitama 3501165 (JP); YOSHIOKA, T., Snow Brand Milk Products Co. Ltd., Kawagoe-shi, Saitama3501165 (JP)
(74) Representative: Wakerley, Helen Rachael
(86) International application number: PCT/JP2005/007158
(87) International publication number: WO 2006/112012

(57) **Abstract**

Provided is a nutrient composition for promoting collagen production, which contains vitamin C, an iron preparation, and collagen as active ingredients, promotes collagen production in a living body such as skin or bone, prevents or treats skin aging or bone and joint diseases, and is an iron preparation-containing composition having stability imparted thereto. Also provided are a food or beverage, a feed, and a medicine each containing the nutrient composition incorporated therein.

## Description

### Technical Field

The present invention relates to a nutrient composition comprising vitamin C, an iron-preparation, and collagen, that promotes collagen production in such body components as skin or bone, preventing or treating skin aging such as skin roughness, wrinkles, and decreased elasticity, or preventing or treating bone and joint diseases such as osteoporosis, bone fracture, arthritis, and rheumatism, which also gives higher stability of iron preparation.

### Background Art

In recent years, studies on skin aging are being made, and it has been confirmed that the skin aging is caused by complex factors such as decreased metabolism due to aging, sunlight (ultraviolet ray), drying, and oxidation. The studies have revealed that those factors cause a significant decrease in the amount of collagen that is the most major matrix component in corium. If a tension-maintaining mechanism such as skin turgor or elasticity maintained by collagen is damaged by action of ultraviolet ray or the like, wrinkles and sags increase in the skin, resulting in aged skin. Further, collagen retains water in its molecule to retain moisture in skin, so when the collagen is damaged, the skin becomes dry and rough. Therefore, it is desired to provide a nutrient composition that promotes a biosynthesis of collagen which is important in constituting a corium layer to prevent the skin from aging, and is safe to eat.

In recent years, as the population ages, patients with bone and joint diseases such as osteoporosis, bone fracture, arthritis, and rheumatism have increased. In order to prevent various bone and joint diseases such as osteoporosis, bone fracture, arthritis, and rheumatism, it is necessary to increase a bone mass per bone volume, that is, a bone matrix including an organic matter such as collagen and a bone mineral including an inorganic matter such as calcium or phosphorus. That is, a decrease in collagen in born due to aging, menopause, etc. may increase the risk of a bone and joint disease such as osteoporosis. Therefore, it is desired to provide a nutrient composition for promoting collagen production that can promote a biosynthesis of collagen in born to prevent or treat a bone and joint disease and is highly safe to eat.

On the other hand, each of vitamin C and iron is known to play an important role in a biosynthesis of collagen. That is, vitamin C and iron are known to be essential for a biosynthesis of hydroxyproline and hydroxylysine that are amino acids specific to collagen (see Non-patent Document 1, for example). Meanwhile, it has been reported that oral intake of collagen can increase the amount of collagen in skin and increase bone density (see Non-patent Documents 2 and 3, for example). However, separate intake of vitamin C and iron or collagen cannot promote a biosynthesis of collagen at a high level. Therefore, it is desired to provide a nutrient composition for promoting collagen production that can increase a collagen biosynthesis level in skin or bone in a more efficient manner.

Meanwhile, in recent years, many iron preparations and iron-enriched foods have been developed, but when an iron preparation is incorporated in an amount sufficient for supplementation of iron, astringent taste or fishy smell of iron is caused, resulting in low palatability. Moreover, when an iron preparation is mixed with a substance having a property of releasing iron or reducing the released iron, such as vitamin C, iron is released and reduced to cause astringent taste of iron, and becomes unstable to form precipitates. Therefore, at present, it is difficult to mix a substance having a property of enhancing the absorbability of iron, such as vitamin C, in iron-enriched foods and drinks.
Non-patent Document 1: Harper's Biochemistry, original 24th edition, edited and translated by Yoshito Kaziro, Maruzen Co., Ltd., p.315, 1997.
Non-patent Document 2 : Ryuj i Asano and two others, BIO INDUSTRY, CMC Publishing CO., LTD., Vol.18, No.4, p.11, 2001.
Non-patent Document 3: Yoshiko Ishimi and two others, Osteoporosis Japan, Life Science Publishing Co., Ltd., Vol. 11, No.2, p.34, 2003.

### Disclosure of the Invention

### Problems to be solved by the Invention

An object of the present invention is to provide: a nutrient composition for promoting collagen production that can promote a biosynthesis of collagen that constitutes a corium layer to prevent the skin from aging and is safe enough to eat; and a food or beverage, a feed, and a medicine each containing the nutrient composition incorporated therein.
Another object of the present invention is to provide: a nutrient composition for promoting collagen production that can promote a biosynthesis of collagen in born to prevent or treat a bone and joint disease such as osteoporosis and is highly safe to eat; and a food or beverage, a feed, and a medicine each containing the nutrient composition incorporated therein.
Still another object of the present invention is to provide: a nutrient composition for promoting collagen production that has suppressed astringent taste of iron or formation of precipitates that is caused by mixing an iron preparation with a substance having a property of releasing iron or reducing the released iron, such as vitamin C; and a food or beverage, a feed, and a medicine each containing the nutrient composition incorporated therein.

### Means for solving the Problems

In view of the above-mentioned problems, the inventors of the present invention have made extensive studies on a component that more effectively promotes in vivo synthesis of collagen, and as a result, have discovered that simultaneous intake of vitamin C, iron, and collagen can effectively increase the amount of collagen in skin or born. Moreover, they have discovered that addition of milk protein to iron preparation can stabilize a composition containing the iron preparation, thereby completing the present invention. Therefore, the present invention have achieved the promotion of collagen production by incorporating vitamin C, an iron preparation, and collagen and the stabilization of an iron preparation by incorporating milk protein, thereby attaining the above-mentioned objects.

Therefore, a feature of the present invention is to promote collagen production by adding vitamin C, an iron preparation, and collagen as active ingredients.

A further feature of the present invention is to provide a nutrient composition containing vitamin C, collagen, and an iron preparation containing milk protein, which has stability imparted by adding milk protein to an iron preparation unstable against a substance having a property of releasing iron or reducing the released iron, such as vitamin C.

Moreover, a further feature of the present invention is to provide a nutrient composition having a collagen production-promoting effect, which includes a raw material or the like that is generally used in a food or drink, a feed, or a medicine in those components and to provide various foods or beverages, feeds, and medicines obtained by incorporating the nutrient composition for promoting collagen production.

### Effect of the Invention

Oral intake of a nutrient composition containing vitamin C, an iron preparation, and collagen of the present invention can promote collagen production in a living body such as skin or bone and can prevent or treat skin aging such as skin roughness, wrinkles, and decreased elasticity, and bone and joint diseases such as osteoporosis, bone fracture, arthritis, and rheumatism. Further, since milk protein is incorporated in an iron preparation, it is possible to provide a stable composition even if the iron preparation is mixed with a substance having a property of releasing iron or reducing the released iron, such as vitamin C.

### Best Mode for carrying out the Invention

A feature of a nutrient composition for promoting collagen production of the present invention is to contain vitamin C, an iron preparation, and collagen as active ingredients.
Examples of the vitamin C include an ascorbic acid derivative such as L-ascorbic acid or sodium L-ascorbate, an ascorbic acid preparation obtained by coating ascorbic acid with an emulsifier or the like, and a mixture containing two or more of those vitamin Cs at an arbitrary rate. In addition, natural products containing vitamin C such as acerola and lemon may also be used.

Meanwhile, examples of the iron preparation include: an inorganic iron such as ferrous sulfate, sodium ferrous citrate, or ferric pyrophosphate; an organic iron such as heme iron, ferritin iron, or lactoferrin iron; and a mixture containing two or more of those irons at an arbitrary rate. In addition, natural products containing iron such as spinach or liver may also be used.

Moreover, examples of the collagen include: an extract obtained by treating bone, skin, or the like of a mammal such as bovine or swine with an acid or alkaline; a peptide obtained by hydrolyzing the extract with a protease such as pepsine, trypsin, or chymotrypsin; and a mixture containing two or more of those collagens at an arbitrary rate.

The nutrient composition for promoting collagen production of the present invention may be a product obtained by only mixing vitamin C, an iron preparation, and collagen serving as active ingredients as they are or may be a product which includes, in addition to those active ingredients, raw materials or the like that are generally used in other foods or beverages, feeds, and medicines such as sugars, lipids, and flavors. It is also possible to provide foods or beverages, feeds, and medicines obtained by incorporating a nutrient composition for promoting collagen production of the present invention in other foods or beverages, feeds, and medicines.
The nutrient composition for promoting collagen production of the present invention may be used to prepare powders, granules, tablets, capsules, drinks, and the like in accordance with a conventional method. In addition, after the preparation step, those products may be incorporated in foods or beverages such as a nutritional supplement, yogurt, lactic beverage, and wafer, feeds, and medicines.

A method of preparing a nutrient composition for promoting collagen production containing vitamin C, an iron preparation, and collagen of the present invention is not particularly limited, but for example, in a case where the composition is prepared in a solution, the method includes the following steps of: suspending or dissolving vitamin C, an iron preparation, and collagen in deionized water; mixing the solution with stirring; and preparing a food or beverage, animal feed, or medicine from the mixture as required. The conditions of mixing with stirring are not particularly limited as long as vitamin C, an iron preparation, and collagen can be mixed sufficiently, and it is possible to perform mixing with stirring using an ultra disperser or the like. In addition, a solution of the nutrient composition for promoting collagen production may be optionally concentrated using an RO membrane or dried by spray-drying, freeze-drying, or the like before use so that the solution can be easily used in foods or beverages, animal feeds, and medicines.

The nutrient composition for promoting collagen production of the present invention can be subjected to a sterilization treatment that is generally used in production processes of foods or beverages, feeds, and medicines, and even if the composition is in the form of powder, it can be subjected to dry-heat sterilization. Therefore, it is possible to prepare foods or beverages, feeds, and medicines in various forms such as liquid, gel, powder, and granule containing a nutrient composition for promoting collagen production of the present invention.

The effective dose of the nutrient composition for promoting collagen production of the present invention varies depending on the age, clinical state, therapeutic effect, etc. An animal experiment using rats revealed that it is necessary to take vitamin C, iron, and collagen in amounts of 10 mg, 0.8 mg, and 100 mg or more, respectively, per kg rat body weight for exerting a collagen production-promoting effect in skin or born. Therefore, in general, intake of vitamin C, iron, and collagen in amounts of 10 mg, 0.8 mg, and 100 mg or more per adult per day can be expected to provide the effect, so those components may be added to a nutrient composition for promoting collagen production so as to achieve those requirements.

Hereinafter, the present invention will be described in detail with reference to Examples and Test Examples, but the present invention is not limited by those examples.

### Example 1

8 g of an iron-saturated lactoferrin solution with an iron content of 1%, 1,000 mg of vitamin C (L-ascorbic acid, manufactured by Tanabe Seiyaku Co., Ltd.), and 11.3 g of collagen (Nippi Peptide PRA, manufactured by Nippi, Inc.) were dissolved in 979.7 g of deionized water, and the solution was mixed with stirring using an ultra-disperser (ULTRA-TURRAX T-25; manufactured by IKA Japan K.K.) at 9,500 rpm for 10 minutes, to thereby yield 1,000 g of a nutrient composition for promoting collagen production of the present invention. The nutrient composition was found to contain vitamin C, iron, and collagen in amounts of 100 mg, 8 mg, and 1,000 mg, respectively, per 100 g.

### Referential Example 1

8 g of an iron-saturated lactoferrin solution with an iron content of 1% and 1,000 mg of vitamin C (L-ascorbic acid, manufactured by Tanabe Seiyaku Co., Ltd.) were dissolved in 991 g of deionized water, and the solution was mixed with stirring using an ultra-disperser (ULTRA-TURRAX T-25; manufactured by IKA Japan K.K.) at 9, 500 rpm for 10 minutes. The solution was found to contain vitamin C and iron in amounts of 100 mg and 8 mg respectively, per 100 g.

### Referential Example 2

11.3 g of collagen (Nippi Peptide PRA, manufactured by Nippi, Inc.) was dissolved in 988.7 g of deionized water, and the solution was mixed with stirring using an ultra-disperser (ULTRA-TURRAX T-25; manufactured by IKA Japan K.K.) at 9,500 rpm for 10 minutes. The solution was found to contain collagen in an amount of 1,000 mg per 100 g.

### Test Example 1

An animal experiment using rats was performed to examine the effect of the nutrient composition for promoting collagen production of the present invention. Seven-week-old Wistar male rats were divided into the following four test groups (10 rats per group) including: a group administered with physiological saline (group A), a group administered with the solution obtained in Referential Example 1 in an amount of 10 g per kg rat body weight (group B), a group administered with the solution obtained in Referential Example 2 in an amount of 10 g per kg rat body weight (group C), and a group administered with the nutrient composition for promoting collagen production obtained in Example 1 in an amount of 10 g per kg rat body weight (group D). The samples were administered to the rats once a day using a probe, and the rats were fed for three weeks.
The amount of skin collagen was determined by: treating the corium of a rat in accordance with the method of Nimni et al. (see M.E.Nimni et al., Arch. Biochem. Biophys., Vol.122, p.292, 1967) ; and measuring the amount of hydroxyproline contained in the soluble fraction. Hydroxyproline is a special amino acid that is contained only in collagen and accounts for about 10% of the total amino acids of collagen, and therefore measurement of the amount of hydroxyproline enables estimating the amount of collagen (see Ryuj i Asano et al., BIO INDUSTRY, Vol. 18, No.4, p. 12, 2001). The results are shown in Table 1.

**[Table 1]**

| Groups | Amount of hydroxyproline (µg/ml) |
|---|---|
| Group A | 0.33 ± 0.05^{a} |
| Group B | 0.46 ± 0.08^{b} |
| Group C | 0.48 ± 0.07^{b} |
| Group D | 0.73 ± 0.09^{c} |

The numeral values represent means ± standard errors. There are significant differences between different alphabets (p<0.05).

Table 1 shows that the amount of hydroxyproline in the soluble fraction of group D after three-week administration is significantly higher compared to groups A, B, and C. The results revealed that simultaneous intake of vitamin C, iron, and collagen enables promoting collagen production in skin compared to separate intake of each of them. Meanwhile, simultaneous intake of vitamin C, iron, and collagen in amounts of 10 mg, 0.8 mg, and 100 mg, respectively, per kg rat body weight was found to provide a collagen production-promoting effect.

### Test Example 2

An animal experiment using rats was performed to examine the effect of the nutrient composition for promoting collagen production of the present invention. Four-week-old SD female rats were preliminarily fed for one week, were subjected to surgery to remove the ovaries, and were fed with a calcium-deficient diet for five weeks. Those rats were divided into the following four test groups (6 rats per group) including: a group administered with physiological saline (group A), a group administered with the solution obtained in Referential Example 1 in an amount of 10 g per kg rat body weight (group B), a group administered with the solution obtained in Referential Example 2 in an amount of 10 g per kg rat body weight (group C), and a group administered with the nutrient composition for promoting collagen production obtained in Example 1 in an amount of 10 g per kg rat body weight (group D). The samples were administered to the rats once a day using a probe, and the rats were fed for three weeks. Note that, during the three weeks, the rats of the respective groups were allowed to freely eat a feed containing 300 mg of calcium, 230 mg of phosphorus, and 50 mg of magnesium. The amount of collagen in born was measured by: hydrolyzing bone with hydrochloric acid; and quantifying hydroxyproline, i.e., special amino acids contained only in collagen, and hydroxylysine. Hydroxylysine is also a special amino acid contained only in collagen, and as in the case of hydroxyproline, measurement of the amount of hydroxylysine enables estimating the amount of collagen. The results of quantification of the amounts of hydroxyproline and hydroxylysine are shown in Table 2.

**[Table 2]**

| Groups | Amount of hydroxyproline (µg/ml) | Amount of hydroxyproline (µg/ml) |
|---|---|---|
| Group A | 20.4 ± 0.6^{a} | 5.9 ± 0.1^{a} |
| Group B | 21.6 ± 0.3^{b} | 6.2 ± 0.2^{b} |
| Group C | 21.7 ± 0.5^{b} | 6.1 ± 0.1^{b} |
| Group D | 23.1 ± 0.9^{c} | 6.8 ± 0.2^{c} |

The numeral values represent means ± standard errors. There are significant differences between different alphabets (p<0.05).

Table 2 shows that the amount of hydroxyproline and hydroxylysine in bone of group D after three-week administration is significantly higher compared to groups A, B, and C. The results revealed that simultaneous intake of vitamin C, iron, and collagen enables promoting collagen production in bone compared to separate intake of each of them. Meanwhile, simultaneous intake of vitamin C, iron, and collagen in amounts of 10 mg, 0.8 mg, and 100 mg, respectively, per kg rat body weight was found to provide a collagen production-promoting effect.

### Example 2

0.4 g of ammonium iron citrate with an iron content of 20% and 30 g of skim milk powder were dissolved in 900 g of deionized water, and the solution was mixed with stirring using an ultra-disperser (ULTRA-TURRAX T-25; manufactured by IKA Japan K. K.) at 9, 500 rpm for 10 minutes. To the solution there were added 1.13 g of vitamin C (L-ascorbic acid, manufactured by Tanabe Seiyaku Co., Ltd.), 11.4 g of collagen (CPB-5, manufactured by JELLICE Co., Ltd.), and 57.07 g of deionized water, to thereby yield 1,000 g of a nutrient composition for promoting collagen production of the present invention. The nutrient composition was found to contain vitamin C, iron, and collagen in amounts of 100 mg, 8 mg, and 1,000 mg, respectively, per 100 g.

### Example 3

29 g of ferric pyrophosphate with an iron content of 28% and 4,000 g of skimmilkpowderwere dissolved in 90 kg of deionized water, and the solution was mixed with stirring using a TK homomixer (MARK II 160, manufactured by PRIMIX Corporation) at 3,600 rpm for 40 minutes. To the solution there were added 106 g of vitamin C (coated ascorbic acid type FC, manufactured by DSM Nutrition Japan K.K.), 1,140 g of collagen (SCP-5000, manufactured by Nitta Gelatin Inc.), and 4,725 g of deionized water, followed by freeze-drying, to thereby yield 5 kg of a nutrient composition for promoting collagen production of the present invention. The nutrient composition was found to contain vitamin C, iron, and collagen in amounts of 2,000 mg, 160 mg, and 20,000 mg, respectively, per 100 g.

### Example 4

80 g of heme iron with an iron content of 1% and 200 g of skim milk powder were dissolved in 90 kg of deionized water, and the solution was mixed with stirring using a TK homomixer (MARK II 160, manufactured by PRIMIX Corporation) at 3,600 rpm for 20 minutes. To the solution there were added 12 g of vitamin C (coated vitamin C, F type, manufactured by BASF Japan Ltd.), 113 g of collagen (Nippi Peptide PRA, manufactured by Nippi, Inc.), and 9,595 g of deionized water, followed by concentrating using an RO membrane, to thereby yield 10 kg of a nutrient composition for promoting collagen production of the present invention. The nutrient composition was found to contain vitamin C, iron, and collagen in amounts of 100 mg, 8 mg, and 1,000 mg, respectively, per 100 g.

### Example 5

8 g of an iron-saturated lactoferrin solution with an iron content of 1% and 20 g of skim milk were dissolved in 600 g of deionized water, and the solution was mixed with stirring using an ultra-disperser (ULTRA-TURRAX T-25; manufactured by IKA Japan K.K.) at 9,500 rpm for 10 minutes. To the solution there were added 1 g of vitamin C (L-ascorbic acid, manufactured by Tanabe Seiyaku Co., Ltd.), 11.3 g of collagen (Nippi Peptide PRA, manufactured by Nippi, Inc.), 40 g of sorbitol, 2 g of an acidulant, 2 g of a flavor, 5 g of pectin, 5 g of a whey protein concentrate, 1 g of calcium lactate, and 304.7 g of water, and the solution was mixed with stirring, to thereby prepare 1,000 g of a nutrient composition for promoting collagen production of the present invention. The nutrient composition was packed in 200-ml Cheerpacks and sterilized at 85°C for 20 minutes, and the packs were sealed, to thereby prepare five packs of a gel-like food including a nutrient compound of the present invention. The prepared gel-like food was found to form no precipitates or the like and have no strange taste in all the packs. Note that the gel-like food including the nutrient compound was found to contain vitamin C, iron, collagen in amounts of 100 mg, 8 mg, and 1,000 mg, respectively, per 100 g.

### Example 6

2 g of an acidulant was dissolved in 600 g of deionized water, and then 0.4 g of ammonium iron citrate with an iron content of 20% and 30 g of skim milk were dissolved therein, followed by mixing with stirring using an ultra-disperser (ULTRA-TURRAX T-25; manufactured by IKA Japan K.K.) at 9,500 rpm for 10 minutes. To the solution there were added 1.13 g of vitamin C (L-ascorbic acid, manufactured by Tanabe Seiyaku Co., Ltd.), 11.4 g of collagen (CPB-5, manufactured by JELLICE Co., Ltd.), 100 g of maltitol, 20 g of reduced starch syrup, 2 g of a flavor, and 233.07 g of deionized water, to thereby prepare 1,000 g of a nutrient composition for promoting collagen production of the present invention. The nutrient composition was packed in 50-ml glass bottles and heat-sterilized at 90°C for 15 minutes, followed by sealing, to thereby prepare 20 bottles of a beverage containing the nutrient composition of the present invention. The prepared beverage was found to form no precipitates or the like and have no strange taste in all the bottles. Note that the nutrient compound was found to contain vitamin C, iron, collagen in amounts of 100 mg, 8 mg, and 1,000 mg, respectively, per 100 g.

### Example 7

5.5 kg of casein, 5 kg of soybean protein, 1 kg of fish oil, 3 kg of perilla oil, 19 kg of dextrin, 6 kg of a mineral mixture, 1.95 kg of a vitamin mixture, 2 kg of an emulsifier, 4 kg of a stabilizer, and 0.05 kg of a flavor were incorporated in 2.5 kg of the nutrient composition prepared in Example 3 (vitamin C: 2, 000 mg%, iron: 160 mg%, collagen: 20, 000 mg%), and the resultant was packed in 200-ml retort pouches, followed by sterilization using a retort sterilizer (a primary pressure vessel, TYPE: RCS-4CRTGN, manufactured by Hisaka Works, Ltd.) at 121°C for 20 minutes, to thereby yield 50 kg of an enteral nutritional supplement. Note that the enteral nutritional supplement was found to contain vitamin C, iron, collagen in amounts of 100 mg, 8 mg, and 1,000 mg, respectively, per 100 g.

### Example 8

12 kg of soybean cake, 14 kg of skim milk powder, 4 kg of soybean oil, 2 kg of corn oil, 23.2 kg of palm oil, 14 kg of corn starch, 9 kg of wheat flour, 2 kg of bran, 5 kg of a vitamin mixture, 2.8 kg of cellulose, and 2 kg of a mineral mixture were incorporated in 10 kg of the nutrient composition prepared in Example 4 (vitamin C: 100 mg%, iron: 8 mg%, collagen: 1,000 mg%), and the resultant was heat-sterilized at 120°C for 4 minutes, to thereby prepare 100 kg of a dog food. The resultant dog food was found to contain vitamin C, iron, collagen in amounts of 10 mg, 0.8 mg, and 100 mg, respectively, per 100 g.

### Industrial Applicability

According to the present invention, it is possible to provide a nutrient composition containing vitamin C, collagen, and an iron preparation that contains milk protein and having stability provided by incorporating milk protein into an iron preparation that is unstable against a substance having a property of releasing iron or reducing the released iron, such as vitamin C. Meanwhile, it is also possible to provide various foods or beverages, feeds, and medicines each having the nutrient composition for promoting collagen production of the present invention.

## Claims

1. A nutrient composition for promoting collagen production, the nutrient composition comprising vitamin C, an iron preparation, and collagen.

2. A nutrient composition according to Claim 1, wherein the iron preparation contains milk protein.

3. A nutrient composition according to Claim 1 or 2, further comprising a raw material or the like that is generally used in a food or beverage, a feed, and a medicine.

4. A food or beverage, a feed, and a medicine, each comprising the nutrient composition according to any one of Claims 1 to 3 incorporated therein.
